# NEW EUROPEAN PATENT SPECIFICATION

(11) **EP 0 278 744 B2**
(45) Date of publication and mention of the opposition decision: **01.10.2003**
(45) Mention of the grant of the patent: 11.08.1993
(21) Application number: 88301108.2
(22) Date of filing: 10.02.1988
(51) Int. Cl.: A61K 7/16

(54) **Dentifrice composition for desensitising sensitive teeth**
Zahnpastazusammensetzung zur Desensibilisierung empfindlicher Zähne
Composition de dentifrice pour désensibiliser les dents sensibles

(30) Priority: 12.02.1987 IT 6709387; 12.11.1987 IT 6796687
(43) Date of publication of application: 17.08.1988
(73) Proprietor: UNILEVER PLC, London EC4P 4BQ (GB); UNILEVER N.V., 3013 AL Rotterdam (NL)
(72) Inventor: Caserio, Domenico, I-20127 Milan (IT)
(74) Representative: Newbould, Frazer Anthony

(56) References cited:
- EP-A- 0 095 871
- EP-A- 0 161 898
- WO-A-85/04098
- GB-A- 1 466 930
- US-A- 3 863 006
- US-A- 3 952 092
- US-A- 4 022 880
- US-A- 4 357 318
- US-A- 4 471 505
- US-A- 4 645 662
- J. Clinical Periodontology, 1983, no. 10, p. 351-363;
- Orchardson, R. (1974) - "Changes in extracellular fluid composition which excite axons": Proceedings of the Physiological Society, Nov. 1974, 14P - 16P;
- R J Jackson : Dentrifices for the treatment of the dentine hypersensitivity, issued in Chapter 27 of the Clinical and Biological Aspects of Dentrifices, edited by G Embery and G Rolla (1992), Oxford Medical Publications.

## Description

This invention relates to a dentifrice composition for desensitising sensitive teeth.

Dental plaque is the major cause of oral disease. Plaque acids attack the tooth enamel leading to caries, and toxins produced by the plaque cause inflamation of the gums (gingivitis) and eventually gum recession and tooth loss (periodontitis). Recession of the gum from the tooth exposes the dentin of the root which can be damaged making the tooth sensitive to tactile and/or thermal stimuli. The resultant pain can make toothbrushing uncomfortable thus leading to inefficient plaque removal and further exacerbation of the problem.

The dentifrice of the present invention has as its object to mitigate the problem of sensitive teeth by providing an improved formulation which has a novel combination of ingredients providing superior effectiveness.

According to the present invention there is provided a dentifrice composition for desensitising sensitive teeth which comprises in combination a water-soluble source of potassium ions selected from the group consisting of potassium nitrate, potassium citrate and potassium bicarbonate and 2,4,4'- trichloro-2'-hydroxy-diphenyl ether (referred to hereinafter by its international non-proprietary name Triclosan).

Potassium salts which are the source of potassium ions are potassium nitrate, potassium citrate, or potassium bicarbonate. US-A-3 863 006 discloses the use of potassium nitrate as a tooth desensitising agent. Potassium citrate has been disclosed in European Patent Application No. 0 095 871 to be effective to reduce the pain associated with sensitive teeth. The use of potassium bicarbonate has been similarly disclosed in International Patent Application WO 85/04098. They act, as do other water-soluble potassium salts which produce potassium ions, directly on exposed tooth dentin. The other active ingredient of the dentifrice of the invention, Triclosan, has proven efficacy against plaque the major cause of gum recession. Thus, the dentifrice of the invention has a two-pronged attack on the problem of sensitive teeth. The independent actions of its two active components result in the product of the invention having a surprising degree of effectiveness which cannot be attained by the use of either component alone. Furthermore, the use of Triclosan gives various advantages compared with other certain anti-bacterial agents since Triclosan does not stain the teeth and does not impart an unpleasant taste to the dentifrice.

In EP-A-0,161,898 dentifrices are disclosed which may contain an anti-microbial agent such as Triclosan to inhibit the growth of dental plaque. Such compositions may optionally contain a water-soluble salt, including a potassium salt. There is no disclosure of toothpastes that contain Triclosan and any of the potassium salts of the present invention for desensitising sensitive teeth. In the Journal of Clinical Periodontology, 1983,10, page 351-363 a review is given of the clinical and in vitro evaluation of various agents for the treatment of dentine hypersensitivity, such agents including potassium nitrate. The authors concluded that lack of understanding of the mode of action of desensitising agent poses considerable difficulties in the choice of the most suitable treatment, and suggest that consideration should be given now to preventing or reducing dentine exposure, as the latter is expected to increase as a clinical problem. They did not suggest any treatment with a combination of certain potassium salts and Triclosan, which according to the present invention was surprisingly found to produce an unexpected desensitising effect.

The potassium salt is used in an amount, calculated as potassium, of from about 0.7% to about 3%, preferably about 1.5% to about 2.3%, by weight of the dentifrice while the Triclosan is used in an amount of from about 0.1% to about 0.5% by weight of the dentifrice.

The dentifrice may contain various other ingredients well known to those skilled in the art. Such ingredients include an humectant, for example, glycerol or sorbitol, an abrasive cleaning agent, such as silica, a binder or thickening agent, such as sodium carboxymethylcellulose, and a detergent, for example, sodium lauryl sulphate.

The dentifrice composition of the invention may also comprise a polyethylene glycol. Polyethylene glycols having a range of molecular weights have already been proposed for dentifrice use. Any suitable amount of the polyethylene glycol may be included such as from about 1% to about 10% by weight of the dentifrice. The inclusion of polyethylene glycols having molecular weights in the range 200 to 1000 have been suggested in, for example, EP-A-220 890 for use in toothpastes containing Triclosan.

A variety of other minor ingredients can also be included such as flavour, preservative, colouring agent, fluoride, buffering agent, sweetening agent, opacifying agent. All such ingredients are used in conventional amounts.

The following are examples of the novel desensitising dentifrice of the invention. Percentages are by weight.

### Example 1

The dentifrice has the following composition.

| Ingredient | % |
|---|---|
| Silica abrasive agent | 4.00 |
| Silica thickening agent | 11.00 |
| Sorbitol syrup (70% solution) | 48.00 |
| Sodium carboxymethylcellulose | 0.75 |
| Sodium lauryl sulphate | 1.70 |
| Potassium nitrate | 5.00 |
| Triclosan | 0.20 |
| Polyethylene glycol (1500) | 5.00 |
| Sodium monofluorophosphate | 0.82 |
| Sodium benzoate | 0.08 |
| Saccharin | 0.20 |
| Titanium dioxide | 0.50 |
| Flavour | 1.00 |
| Dyestuff | 0.05 |
| Water | to 100.00 |

### Example 2

A dentifrice is made having the composition of Example 1 except that the potassium nitrate is replaced by 5.35g of potassium citrate monohydrate.

### Example 3

A dentifrice is made having the composition of Example 1 except that the potassium nitrate is replaced by 4.95g of potassium bicarbonate.

When the dentifrices of the above Examples are applied to the teeth in the conventional way, such as by brushing, they are effective to reduce the sensitivity of hypersensitive areas of the teeth, especially that of the tooth dentine.

## Claims

1. A dentifrice composition for desensitising sensitive teeth, **characterised in that** it comprises in combination 0.7% to 3% (calculated as potassium) by weight of the dentifrice composition of a water-soluble source of potassium ions selected from the group consisting of potassium nitrate, potassium citrate, and potassium bicarbonate, and 2,4,4'-trichloro-2'-hydroxy-diphenyl ether, together with usual dentifrice ingredients.

2. A dentifrice composition as claimed in Claim 1, **characterised in that** the source of potassium ions is potassium nitrate.

3. A dentifrice as claimed in Claim 1 **characterised in that** the source of potassium ions is potassium citrate.

4. A dentifrice composition as claimed in any of Claims 1 to 3, **characterised in that** it comprises about 0.1 to about 0.5% by weight of 2,4,4'-trichloro-2'-hydroxy-diphenyl ether.

## Patentansprüche

1. zahnputzmittelzusammensetzung zur Desensibilisierung empfindlicher Zähne, **dadurch gekennzeichnet, daß** sie eine Kombination aus 0,7 Gew.% bis 3 Gew.% (gerechnet als Kalium) der Zahnputzmittelzusammensetzung einer wasserlöslichen Kaliumionenquelle, ausgewählt aus der Gruppe von Kaliumnitrat, Kaliumcitrat und Kaliumbicarbonat, und 2,4,4'-Trichlor-2'-hydroxydiphenylether zusammen mit üblichen Zahnputzmittelbestandteilen enthält.

2. Zahnputzmittelzusammensetzung nach Anspruch 1, **dadurch gekennzeichnet, daß** die Kaliumionenquelle Kaliumnitrat ist.

3. Zahnputzmittel nach Anspruch 1, **dadurch gekennzeichnet, daß** die Kaliumionenquelle Kaliumcitrat ist.

4. zahnputzmittelzusammensetzung nach einem der Ansprüche 1 bis 3, **dadurch gekennzeichnet, daß** sie etwa 0.1 bis etwa 0.5 Gew.% 2,4,4'-Trichlor-2'-hydroxy-diphenylether enthält.

## Revendications

1. composition de dentifrice pour désensibiliser les dents sensibles, **caractérisée en ce qu'**elle comprend en combinaison, de 0,7 % à 3% en poids (calculé en tant que potassium) d'une composition de dentifrice d'une source d'ions potassium solubles dans l'eau sélectionnés à partir du groupe composé du nitrate de potassium, du citrate de potassium, et du bicarbonate de potassium et de 2,4,4'-trichloro-2'-hydroxydiphényle éther, avec les ingrédients de dentifrice courants.

2. Composition de dentifrice selon la revendication 1, **caractérisée en ce que** la source d'ions potassium est du nitrate de potassium.

3. Dentifrice selon la revendication 1, **caractérisé en ce que** la source d'ions potassium est du citrate de potassium.

4. Composition de dentifrice selon l'une quelconque des revendications 1 à 3, **caractérisée en ce qu'**elle comprend environ 0,1 à environ 0,5 % en poids de 2,4,4' - trichloro - 2' - hydroxy - diphényle éther.
